(19) Europäisches Patentamt

European Patent Office

Office européen des brevets

(11) **EP 0 706 368 B1**

(12) **FASCICULE DE BREVET EUROPEEN**

(45) Date de publication et mention
de la délivrance du brevet:
**18.10.2000 Bulletin 2000/42**

(21) Numéro de dépôt: **94920528.0**

(22) Date de dépôt: **29.06.1994**

(51) Int. Cl.⁷: $A61K\ 7/48$

(86) Numéro de dépôt international:
**PCT/FR94/00786**

(87) Numéro de publication internationale:
**WO 95/01159 (12.01.1995 Gazette 1995/03)**

(54) **COMPOSITION ANTIACNEIQUE CONTENANT UN EXTRAIT DE PORIA COCOS WOLF**

ANTIAKNE-PRÄPARAT DAS EIN EXTRAKT DES PORIA COCOS WOLF ENTHALT

ANTI-ACNE COMPOSITION CONTAINING A PORIA COCOS WOLF EXTRACT

(84) Etats contractants désignés:
**BE CH DE ES FR GB IT LI**

(30) Priorité: **30.06.1993 FR 9307967**

(43) Date de publication de la demande:
**17.04.1996 Bulletin 1996/16**

(73) Titulaire: **LVMH RECHERCHE
75008 Paris (FR)**

(72) Inventeurs:
• **MEYBECK, Alain
F-92400 Courbevoie (FR)**
• **BONTE, Frédéric
F-92400 Courbevoie (FR)**

(74) Mandataire: **Portal, Gérard et al
Cabinet Beau de Loménie
158, rue de l'Université
75340 Paris Cédex 07 (FR)**

(56) Documents cités:
**US-A- 3 010 878        US-A- 3 369 032**

• **PATENT ABSTRACTS OF JAPAN vol. 9, no. 218 (C-301) (1941) 5 Septembre 1985 & JP,A,60 078 910 (KANEBO K.K.) 5 Mai 1985**
• **PATENT ABSTRACTS OF JAPAN vol. 10, no. 131 (C-346) 15 Mai 1986 & JP,A,60 258 104 (INAHATA KOURIYOU) 20 Décembre 1985**

**Description**

La présente invention concerne essentiellement l'utilisation d'un extrait de champignons Poria cocos Wolf pour la préparation d'une composition cosmétique ou pharmaceutique, notamment dermatologique pour le traitement de l'acné ou des peaux passes, et un procédé de traitement cosmétique.

Il est connu par l'article paru dans la Revue Bulletin de la Société Chimique de France (1980, n°9-10, p. 473-477) l'utilisation d'extraits de champignons Poria cocos Wolf, et plus particulièrement de triterpènes contenus dans ces extraits pour une activité cytotoxique pour le traitement de tumeurs.

On connaît également, à partir de la base de données Excerpta Medica un abrégé en langue anglaise de la revue japonaise Japan Journal of Pharmacology (1992), volume 59 (1), p. 89-96, une activité antinéphrétique d'extraits de champignons Poria cocos Wolf.

On connaît également par la publication de la demande japonaise sous le numéro JP-A-1/038010 POLA une composition cosmétique pour promouvoir la restauration de la chevelure et la croissance des cheveux, contenant parmi un certain nombre de substances actives, un extrait organique, en particulier alcoolique, de champignons Poria cocos Wolf.

On connaît encore par la publication de la demande japonaise sous le numéro JP-A-60/078910 KANEBO une composition cosmétique pour traiter les peaux sèches, améliorer son hydratation, en ayant ainsi un effet embellissant de la peau, contenant un extrait de Bukuryo dénommé Sclérothium de Poria cocos.

Ainsi, ce document KANEBO divulgue essentiellement une activité d'hydratation, qui constitue une activité totalement différente du traitement de l'acné ou des peaux passes, objet de la présente invention.

Encore, le document US-A-3 369 032 enseigne que certains composants des Porias ont un effet anti-acnéique, mais ce document n'enseigne pas l'emploi d'un extrait direct des fongi Poria cocos.

Il en est de même dans le cas du document US-A-3 010 878.

Dans le cadre de la présente invention, il a été découvert de manière inattendue que des extraits organiques ou hydro-organiques de champignons Poria cocos Wolf présentaient une nouvelle activité anti-acnéique et anti-peaux passes.

La présente invention a ainsi pour but de résoudre le nouveau problème technique consistant en la fourniture d'une solution permettant de réaliser de nouvelles formulations cosmétiques ou pharmaceutiques, notamment dermatologiques, à activité anti-acnéique et/ou anti-peaux passes d'une manière simple, reproductible, peu coûteuse, utilisable à l'échelle industrielle et cosmétique et/ou pharmaceutique.

La présente invention résoud pour la première fois ce nouveau problème technique d'une manière satisfaisante.

Ainsi, selon un premier aspect, la présente invention concerne l'utilisation d'un extrait organique ou hydro-organique de champignons Poria cocos Wolf comme agent cosmétique pour le soin des peaux passes ou à tendance acnéique ou pour la fabrication d'une composition et/ou pharmaceutique, notamment dermatologique, pour le traitement de l'acné et des peaux grasses.

On entend dans le cadre de l'invention par l'expression "extrait hydro-organique", un extrait obtenu au moyen d'un mélange d'eau et d'un solvant organique miscible à l'eau. En particulier, dans le cadre de la présente invention, il s'agit de préférence d'un mélange hydro-alcoolique, tel qu'un mélange hydro-éthanolique ou hydro-méthanolique, contenant encore de préférence au moins 50 % en poids d'alcool par rapport au poids total du mélange hydro-alcoolique.

Selon un mode de réalisation particulier, il s'agit d'un extrait alcoolique, en particulier éthanolique total, ou d'un extrait hydro-alcoolique, tel que hydro-éthanolique ou hydro-méthanolique, de Poria cocos Wolf.

Selon un autre mode de réalisation particulier, la concentration en extrait de Poria cocos est comprise entre 0,001 et 5 % en poids par rapport au poids total de la composition finale.

Selon encore un autre mode de réalisation particulier, l'extrait précité est au moins partiellement incorporé dans des phases lamellaires lipidiques hydratées ou dans des liposomes.

Selon encore un autre mode de réalisation particulier, l'extrait précité de Poria cocos est combiné à au moins une autre substance active, de préférence choisie parmi le groupe consistant d'une substance séborégulatrice, d'une substance antiseptique, d'une substance anticomédon.

Selon encore un autre mode de réalisation particulier, la substance séborégulatrice précitée est un extrait de réglisse glycyrrhyza glabra.

Selon encore un autre mode de réalisation particulier, la substance antiseptique précitée est une substance anti-coryne bactérium, telle que le diiséthionate d'héxamidine disponible dans le commerce, un extrait d'isodon japonicus également disponible dans le commerce, la clindamycine, ou l'érythromycine.

Selon encore un autre mode de réalisation particulier, la substance anticomédon précitée est choisie parmi le groupe consistant de la vitamine A acide, de la vitamine A et de ses dérivés, tels que l'acétate, palmitate, propionate, et de l'acide azélaïque.

Selon encore un autre mode de réalisation particulier, la substance est choisie parmi le groupe consistant du glycyrrhyzinate d'ammonium, de l'acide glycyrrhétinique, de l'α-bisabolol, du phosphate de tocophérol.

Selon un deuxième aspect, la présente invention concerne également un procédé de traitement cosmétique des peaux passes, caractérisé en ce qu' on applique topiquement sur les zones grasses concernées de la peau, une quantité cosmétiquement efficace d'un extrait organique ou hydro-organique de champignons Poria cocos Wolf, généralement dans un excipient cosmétiquement acceptable.

Selon une réalisation particulière, on applique une composition cosmétique contenant de 0,001 à 5 % en poids de l'extrait précité de Poria cocos Wolf.

Selon une réalisation particulière, on applique une composition cosmétique contenant un extrait alcoolique, en particulier éthanolique total, ou d'un extrait hydro-alcoolique, tel que hydro-éthanolique ou hydro-méthanolique, de champignons Poria cocos Wolf.

Selon une réalisation particulière, l'extrait précité de Poria cocos Wolf est au moins partiellement incorporé dans des phases lamellaires hydratées ou dans des liposomes.

D'autres buts, caractéristiques et avantages de l'invention apparaîtront à la lumière de la description explicative qui va suivre faite en référence à divers exemples de réalisation ainsi qu'à des essais d'activité, donnés simplement à titre d'illustration et qui ne sauraient donc en aucune façon limiter la portée de l'invention.

Dans l'un ou l'autre des aspects précédents de l'invention, on peut utiliser comme solvant organique d'extraction, de préférence un extrait alcoolique, en particulier choisi parmi le poupe consistant du méthanol, de l'éthanol, du butylèneglycol et du propylèneglycol. Ces solvants peuvent avantageusement être utilisés à l'état pur seuls ou en mélange. On peut également les utiliser en mélange avec de l'eau. Certains extraits sont disponibles dans le commerce et notamment un extrait butylèneglycol de Poria cocos Wolf commercialisé par la société japonaise Maruzen sous le nom de Hoelen BG.

On notera également pour l'un quelconque des aspects précédents de l'invention, que l'extrait précité peut être formulé de manière classique dans des compositions cosmétiques ou pharmaceutiques, notamment dermatologiques, par exemple sous forme de gel, de crème, d'émulsion, de lait, en vue du traitement des peaux passes ou acnéiques, ou pour le traitement de l'acné.

Dans la description, en particulier les exemples, et dans les revendications, les pourcentages sont donnés en poids, sauf indication contraire.

Exemple 1

Fabrication d'un extrait éthanolique de champignons Poria cocos Wolf

A partir de champignons Poria cocos Wolf disponibles dans le commerce, on réalise un broyage de cette matière première afin d'augmenter la surface de contact avec le solvant. On extrait à 40°C avec de l'éthanol la matière première en utilisant une proportion de matière première/solvant de 20 à 100 g/l. Puis l'extrait est concentré sous vide.

Cet extrait est dénommé extrait $I_1$ de l'invention.

Exemple 2

Mise en évidence de l'activité inhibitrice de l'enzyme 5a-réductase d'un extrait de Poria cocos Wolf, enzyme impliquée dans l'acné et les peau grasses

Pour réaliser cet essai, on se base sur la méthode d'inhibition de l'enzyme 5a-réductase qui transforme la testostérone en dihydrotestostérone, décrite dans la littérature dans la revue J. I. D. (1987), 89, p. 87-92.

Il est bien connu à l'homme de l'art que la formation de dihydrotestostérone à partir de testostérone, notamment sous l'action de l'enzyme 5a-réductase, est impliquée dans l'acné et les peaux grasses et qu'une inhibition de cette enzyme permet de lutter efficacement contre l'acné et les pcaux grasses. Ainsi, la mise en évidence sur une substance active quelconque d'une inhibition significative de l'activité de l'enzyme 5a-réductase est un test approprié pour déterminer une activité antiacnéique et anti-peaux grasses. En outre, ce test est reconnu par l'homme de l'art comme étant fiable dans des essais réalisés in vitro selon la méthode décrite dans ladite littérature.

Pour réaliser ce test, et conformément à la littérature, on part d'une lignée de fibroblastes humains normaux de prépuces.

On réalise une culture de cette lignée de fibroblastes dans un milieu E 199C (TECHGEN, France) auquel il a été ajouté 1 % de sérum de veau foetal, à raison de 10 000 cellules de fibroblastes par puits d'une microplaque. A chacun de ces puits, on a également ajouté 0,1 µCi par puits de testostérone tritiée pour une identification par mesure de la radioactivité des métabolites formés au contact des fibroblastes.

Certains puits servent de témoins et ne reçoivent que 1 ml de solution à 0,1 % de DMSO sans substance active. D'autres puits reçoivent la substance active à tester, ici un extrait éthanolique de Poria cocos Wolf tel qu'obtenu à l'exemple 1, dans 1 ml de solution à 0,1 % de DMSO (DiMéthylSulfOxyde).

Dans chacun des cas, respectivement témoin ou substance active à tester. Le temps de contact avec la culture est de 24 h.

Après 24 h, on récupère le surnageant, on extrait les stéroïdes par un mélange de solvant d'extraction acétate d'éthyle-cyclohexane (1/1 en volume) à raison de 1 ml de ce mélange. Les stéroïdes extraits sont déposés sur une plaque de chromatographie en couche mince disponible dans le commerce référence Kieselgel 60 F 254 DC Alu disponible chez Merck®. On utilise comme système éluent de ladite plaque le mélange chloroforme-méthanol 98/2 en volume/volume.

On effectue une lecture avec un scanner adapté à recevoir des plaques de chromatographie sur couches minces de chez Berthold, France. On réalise un comptage de la radioactivité des taches correspondant à la testostérone et à la dihydrotestostérone.

On obtient les résultats suivants répertoriés au tableau I.

TABLEAU I

| Produit | Testostérone | Dihydrotestostérone | Activité A (%) |
|---|---|---|---|
| Témoin (DMSO à 0,1 %) | 47 ± 3 % | 28 ± 1 % | 0 |
| Produit $I_1$ de l'invention de l'exemple 1 (50 µg/ml) | 61 ± 4 % | 20 ± 1 % | 29 % |

L'activité A est déterminée par la formule suivante :

$$A = \frac{Tr - Te}{Te} \times 100$$

dans laquelle :

- Tr représente le pourcentage de testostérone mesuré après 24 h de culture de l'éthantillon traité avec le produit de l'exemple 1 (extrait éthanolique selon l'invention de Poria cocos Wolf dans le DMSO à 0,1 %);
- Te est le pourcentage de testostérone mesuré dans l'échantillon témoin ne recevant que 0,1 % de DMSO, également après 24 h de culture ;

Il résulte clairement du tableau 1 ci-dessus que l'extrait alcoolique de champignons Poria cocos Wolf présente une activité inhibitrice significative de l'enzyme 5a-réductase inhibant de manière correspondante la transformation de testostérone en dihydrotestostérone en rendant ainsi cet extrait particulièrement utile pour le traitement de l'acné et des peaux passes.

Exemple 3

Mise en évidence chez l'homme de l'activité séborégulatrice des compositions selon l'invention

Il est bien connu que les peaux passes, même non-acnéiques, présentent en raison d'une production excessive de sébum un aspect luisant et disgracieux.

La présente expérimentation chez l'homme a pour but de montrer l'activité des compositions de l'invention dans la régulation de la sécrétion de sébum.

Trois préparations ont été essayées, dont la composition figure au tableau II ci-dessous.

La préparation B est une brume, la préparation G est un gel nettoyant et la préparation S est un soin régulateur matifiant.

TABLEAU II

| Composition des préparations testées (pourcentages en poids) | | | |
|---|---|---|---|
| | B (brume) | G (gel) | S (soin) |
| - Extrait butylène-glycol de Poria cocos Wolf du commerce (Hoelen BG) | 0,3 | 0,06 | 3,6 |
| - Extrait d'isodon Japonicus du commerce | 0,15 | 0,03 | 1,8 |

TABLEAU II (suite)

| Composition des préparations testées (pourcentages en poids) | | | |
|---|---|---|---|
| | B (brume) | G (gel) | S (soin) |
| - Extrait hydro-éthanolique de Scutellaria baïcalensis (Ichimaru Pharcos) | 0,05 | 0,01 | 0,6 |
| - Amidon de maïs | 0 | 0 | 8,- |
| - Excipient cosmétique | qsp100,- | qsp 100,- | qsp 100,- |

L'expérimentation est réalisée sur 12 volontaires d'âge moyen 33 ans, présentant une peau passe non-acnéique.

1 - Evaluation de l'effet matifiant

Avant le début de l'expérimentation, une période de trois jours est observée au cours de laquelle aucun soin cosmétique n'est utilisé.

L'effet matifiant de la préparation du soin régulateur S est évalué après une application en hémivisage d'une quantité standardisée à la seringue.

Des mesures de la séborrhée et de la brillance de la peau sont effectuées respectivement au moyen d'un sébumètre et d'un chromamètre.

a - Sébumétrie

On utilise un sébumètre SM 810 Pc disponible dans le commerce chez COURAGE et KHAZAKA.

Des mesures du taux des lipides à la surface cutanée ont été réalisées par sébumétrie au niveau du front. Le niveau courant et le débit d'excrétion sébacée mesurés 30 min après dégraissage sont exprimés en indices lipidiques.

Les mesures du niveau courant ont été réalisées avant applications et toutes les 2 h au cours d'une période de 8 h pour observer la cinétique.

Les résultats comparatifs exprimés en indices lipidiques figurent au tableau III.

TABLEAU III

| Etude cinétique de la séborrhée après traitement | | |
|---|---|---|
| | côté non traité | côté traité |
| Temps zéro (application) | $6,83 \pm 3,71$ | $7,50 \pm 3,86$ |
| 2 h après application | $117,70 \pm 46,13$ | $88,67 \pm 34,21$ |
| 4 h après application | $165,40 \pm 39,33$ | $147,83 \pm 42,01$ |
| 6 h après application | $203,90 \pm 22,88$ | $176,63 \pm 26,63$ |
| 8 h après application | $213,67 \pm 30,00$ | $181,70 \pm 35,21$ |

La différence entre les indices lipidiques obtenus sur la zone témon non traitée et sur la zone traitée est significative à 2 h, et se maintient à 4 h, 6 h et 8 h.

Il ressort clairement du tableau III que l'application du soin régulateur S selon l'invention a permis de diminuer significativement la vitesse de regraissage de la peau après nettoyage. Le niveau courant maximum est atteint en 6 h.

b - Chromamétrie

La luminance de la peau a été mesurée au niveau de l'angle nasogénien par chromamétrie au moyen du Chromamètre Minolta CR 200 avant application d'une part, et toutes les 2 h pendant 8 h d'autre part, sur une joue traitée et sur une joue témoin.

Les résultats comparatifs de luminance de la peau sont rapportés au tableau IV ci-après.

TABLEAU IV

| Luminance de la peau, après traitement | | |
|---|---|---|
| | côté non traité | côté traité |
| Temps zéro (application) | 62,41 ± 2,19 | 62,60 ± 1,88 |
| 2 h après application | 62,31 ± 1,93 | 62,16 ± 2,22 |
| 4 h après application | 62,28 ± 1,71 | 61,79 ± 1,59 |
| 6 h après application | 62,09 ± 1,73 | 61,41 ± 1,60 |
| 8 h après application | 62,25 ± 1,82 | 61,73 ± 2,10 |

Sur le côté traité le paramètre de luminance L accuse une diminution constante durant les 6 premières heures.

Ces évolutions sont toutes statistiquement significatives par rapport à la valeur correspondant au moment de l'application de la préparation de l'invention (temps zéro).

En conclusion, ces essais démontrent clairement que la préparation selon l'invention présente un effet immédiat, dès sa première application, sur la diminution de l'écoulement excessif de sébum et sur l'atténuation de la brillance de la peau, par rapport à des zones de peau non traitées.

2 - Amélioration de l'état de la peau

Les trois préparations B, G et S sont appliquées deux fois sur le front par jour. Un nettoyage est effectué avec le gel G, suivi d'une pulvérisation de la brume B, puis de l'application du soin S. Ce traitement est suivi pendant 30 jours.

L'amélioration de l'état de la peau des 12 volontaires, présentant tous une peau passe non-acnéique, est évaluée avant, au cours et à la fin d'une période de traitement de 30 jours.

Une première amélioration est faite après 15 jours de traitement, une autre est faite après 30 jours.

L'intensité de la séborrhée est repérée par rapport à quatre niveaux :

niveau zéro : pratiquement pas de séborrhée
niveau 1 : séborrhée de faible intensité
niveau 2 : séborrhée de moyenne intensité
niveau 3 : séborrhée de forte intensité.

Le tableau V ci-après rassemble les observations sur les 12 sujets :

TABLEAU V

| Répartition des sujets traités avant, au cours et en fin de traitement en fonction de l'intensité de séborrhéee | | | |
|---|---|---|---|
| | Avant utilisation | après 15 jours d'utilisation | après 30 jours d'utilisation |
| Niveau 0 | 0 | 0 | 0 |
| Niveau 1 | 0 | 4 | 9 |
| Niveau 2 | 10 | 8 | 3 |
| Niveau 3 | 2 | 0 | 0 |

Nous observons un déplacement global de l'effectif, des classes de forte intensité vers les classes de faible intensité : disparition dès 15 jours des sujets en niveau 3, diminution progressive au cours de l'essai de l'effectif en niveau 2 et apparition du niveau 1, majoritaire en fin d'essai.

Une analyse plus fine des résultats individuels au cas par cas, en considérant également les évolutions intra-niveaux, indique :

- après 15 jours de traitement,

    Etat stationnaire : 2 cas
    Diminution d'un niveau : 5 cas
    Diminution légère (intra-niveau) : 5 cas

- après 30 jours de traitement

    Etat stationnaire : 1 cas
    Diminution d'un niveau : 8 cas
    Diminution légère (intra-niveau) : 3 cas.

Ces résultats cliniques, portant sur l'appréciation de l'intensité du niveau courant de sébum (avant dégraissage pour les mesures de débit), sont très favorables au traitement : en fin d'essai, 8 sujets sur 12 se présentent avec un niveau de séborrhée notablement diminué. L'amélioration est moins sensible chez 3 sujets, et seul un cas ne semble pas avoir été affecté par le traitement.

Ainsi, les préparations selon l'invention présentent chez l'homme une très nette efficacité pour traiter les peaux passes en régulant la production et l'écoulement du sébum. Leur utilisation contribue de ce fait à améliorer l'état de la peau. Celle-ci devient plus saine et d'aspect plus esthétique.

On donne ci-après divers exemples de compositions cosmétiques ou pharmaceutiques, notamment dermatologiques utilisant un extrait de Poria cocos Wolf selon la présente invention.

Exemple 4

Composition cosmétique sous forme de gel traitant

Cette composition cosmétique présente les ingrédients suivants en poids :

| | |
|---|---|
| -extrait butylèneglycol de Poria cocos Wolf du commerce Hoelen BG | 0,5 g |
| -extrait d'isodon (antiseptique) | 0,5 g |
| -glycyrrhizinate d'ammonium comme substance anti-inflammatoire | 0,3 g |
| -Crémophor RH 40[®] | 1 g |
| -Carbopol 940[®] | 1 g |
| -excipient aqueux parfumé avec conservateur | q.s.p. 100 g |

Les extraits sont ajoutés avec le Crémophor RH 40[®] à de l'eau pour former 50 % de la composition, qui sont ajoutés à un gel de Carbopol à 2 % contenant parfums et conservateurs, en formant ainsi la composition finale de formule ci-dessus.

On applique ce gel deux fois par jour contre les points noirs pour les peaux à tendance acnéique jusqu'à obtention d'une bonne qualité de la peau, soit trois semaines environ.

Exemple 5

Composition cosmétique sous forme de lotion nettoyante

Cette composition présente les ingrédients suivants :

| | |
|---|---|
| -extrait éthanolique Poria cocos de l'exemple 1 | 0,1 g |

(suite)

| | |
|---|---|
| -diiséthionate d'hexamidine | 0,1 g |
| -acide hyaluronique (substance hydratante) | 0,1 g |
| -glycérine | 0,2 g |
| -excipient aqueux parfumé contenant un conservateur | q.s.p. 100 g |

On mélange l'ensemble des ingrédients jusqu'à obtention d'une lotion homogène que l'on utilisera pour un nettoyage bi-quotidien des peaux grasses.

Cette lotion est appliquée notamment le soir, sur les zones de peaux passes, après le démaquillage éventuel pour une prévention et/ou le traitement de cette peau.

<u>Exemple 6</u>

<u>Composition dermatologique anti-acné</u>

Cette composition présente les ingrédients suivants :

| | |
|---|---|
| - acide rétinoïque | 0,05g |
| - Extrait I$_1$ de Poria cocos de l'exemple 1 | 0,5 g |
| - Phosphate dc clindamycine | 1 g |
| - Propylèneglycol | 5 g |
| - Ethanol | 30 g |
| - Excipient gélifié avec Carbopol 940® et conservateur q.s.p | 100 g |

Pour préparer cette composition, on dissout tout d'abord les différents ingrédients cités dans l'éthanol que l'on ajoute ensuite dans l'excipient gélifié. Cette composition peut être utilisée en tamponnant localement sur les lésions acnéiques jusqu'à obtention d'un effet de disparition de ces lésions acnéiques.

**Revendications**

1.    Utilisation cosmétique d'un extrait organique ou hydro-organique de champignons poria cocos Wolf comme agent actif pour le soin des peaux grasses ou à tendance acnéique.

2.    Utilisation selon la revendication 1, caractérisée en ce que l'extrait précité de Poria cocos est combiné à au moins une autre substance active, de préférence choisie parmi le groupe consistant en une substance séborégulatrice, une substance antiseptique, une substance anticomédon.

3.    Utilisation selon la revendication 2, caractérisée en ce que ladite substance est une substance séborégulatrice, de préférence un extrait de réglisse glycyrrhyza glabra.

4.    Utilisation selon la revendication 2, caractérisée en ce que ladite substance est une substance à activité antiseptique, de préférence une substance à activité anti-coryne bactérium, telle qu'un extrait d'isodon japonicus.

5.    Utilisation selon la revendication 2, caractérisée en ce que ladite substance est une substance à activité anticomédon choisie parmi le groupe consistant de la vitamine A acide, de la vitamine A et de ses dérivés, tels que l'acétate, le palmitate, le propionate et de l'acide azélaïque.

6.    Utilisation selon la revendication 2, caractérisée en ce que ladite substance est choisie parmi le groupe consistant du glycyrrhizinate d'ammonium, de l'acide glycyrrhétinique, de l'α-bisabolol, du phosphate de tocophérol

**7.** Utilisation d'un extrait organique ou hydro-organique de champignons poria cocos Wolf pour la fabrication d'une composition pharmaceutique, notamment dermatologique, pour le traitement de l'acné et des peaux grasses.

**8.** Utilisation selon la revendication 7, caractérisée en ce que l'extrait précité de Poria cocos est combiné à au moins une autre substance active, de préférence choisie parmi le groupe consistant d'une substance séborégulatrice, d'une substance antiseptique, d'une substance anticomédons, d'une substance anti-inflammatoire.

**9.** Utilisation selon la revendication 8, caractérisée en ce que la substance séborégulatrice précitée est un extrait de réglisse glycyrrhyza glabra.

**10.** Utilisation selon la revendication 8, caractérisée en ce que la substance antiseptique précitée est une substance anti-coryne bactérium, telle que diiséthionate d'hexamidine, un extrait d'isodon japonicus, la clindamycine, ou l'érythromycine.

**11.** Utilisation selon la revendication 8, caractérisée en ce que la substance anticomédon précitée est choisie parmi le groupe consistant de la vitamine A acide, de la vitamine A et de ses dérivés, telle que l'acétate, palmitate, propionate et de l'acide azélaïque.

**12.** Utilisation selon la revendication 8, caractérisée en ce que la substance anti-inflammatoire précitée est choisie parmi le groupe consistant du glycyrrhizinate d'ammonium, de l'acide glycyrrhétinique, de l'$\alpha$-bisabolol, du phosphate de tocophérol et d'un corticoïde.

**13.** Utilisation selon l'une des revendications 1 à 12, caractérisée en ce qu'il s'agit d'un extrait alcoolique, en particulier éthanolique total, ou d'un extrait hydro-alcoolique, tel que hydro-éthanolique ou hydro-méthanolique, de Poria cocos Wolf.

**14.** Utilisation selon la revendication 1 à 13, caractérisée en ce que la concentration en extrait de Poria cocos est comprise entre 0,001 et 5 % en poids par rapport au poids total de la composition finale.

**15.** Utilisation selon une des revendications 1 à 14, caractérisée en ce que l'extrait précité est au moins partiellement incorporé dans des phases lamellaires lipidique hydratées ou dans des liposomes.

**16.** Procédé de traitement cosmétique des peaux grasses, caractérisé en ce qu'on applique topiquement sur les zones grasses concernées de la peau, une quantité cosmétiquement efficace d'un extrait organique ou hydro-organique de champignons Poria cocos Wolf, seul ou inclus dans un excipient cosmétiquement acceptable.

**17.** Procédé selon la revendication 16, caractérisé en ce qu'on applique une composition cosmétique contenant de 0,001 à 5 % en poids de l'extrait précité de Poria cocos Wolf.

**18.** Procédé selon la revendication 16 ou 17, caractérisé en ce qu'on applique une composition cosmétique contenant un extrait alcoolique, en particulier éthanolique total, ou d'un extrait hydro-alcoolique, tel que hydro-éthanolique ou hydro-méthanolique, de champignons Poria cocos Wolf.

**19.** Procédé selon une des revendications 16 à 18, caractérisé en ce que l'extrait précité de Poria cocos Wolf est au moins partiellement incorporé dans les phases lamellaires lipidiques hydratées ou dans des liposomes.

**20.** Procédé selon une des revendications 16 à 19, caractérisé en ce que l'extrait précité de Poria cocos est combiné à au moins une autre substance active, de préférence choisie parmi le groupe consistant en une substance séborégulatrice, une substance antiseptique, une substance anticomédon.

**21.** Procédé selon la revendication 20, caractérisé en ce que ladite substance est une substance séborégulatrice constituant un extrait de réglisse glycyrrhyza glabra.

**22.** Procédé selon la revendication 20, caractérisé en ce que ladite substance est une substance à activité antiseptique, de préférence une substance à activité anti-coryne bacterium, telle qu'un extrait d'isodon japonicus.

**23.** Procédé selon la revendication 20, caractérisé en ce que ladite substance est une substance à activité anticomédon choisie parmi le groupe consistant de la vitamine A acide et de ses dérivés, tels que l'acétate, le palmitate ou le propionate, et de l'acide azélaïque.

24. Procédé selon la revendication 20, caractérisé en ce que ladite substance est choisie parmi le groupe consistant du glycyrrhizinate d'ammonium, de l'acide glycyrrhétinique, de l'α-bisabolol, du phosphate de tocophérol.

**Claims**

1. Cosmetic use of an organic or hydro-organic extract of Poria cocos Wolf fungi as active agent for the care of oily skins or skins inclined to be acneic.

2. Use according to claim 1, characterised in that the above-mentioned Poria cocos extract is combined with at least one other active substance, preferably selected from the group consisting of a seboregulatory substance, an antiseptic substance, and an anti-comedo substance.

3. Use according to claim 2, characterised in that said substance is a seboregulatory substance, preferably an extract of licorice, glycyrrhyza glabra.

4. Use according to claim 2, characterised in that said substance is a substance having antiseptic activity, preferably a substance having anti-corynebacterium activity, such as an isodon japonicus extract.

5. Use according to claim 2, characterised in that said substance is a substance having anti-comedo activity selected from the group consisting of vitamin A acid, vitamin A and its derivatives such as the acetate, palmitate, propionate, and azelaic acid.

6. Use according to claim 2, characterised in that said substance is selected from the group consisting of ammonium glycyrrhizinate, glycyrrhetinic acid, α-bisabolol, tocopherol phosphate.

7. Use of an organic or hydro-organic extract of Poria cocos Wolf fungi for the preparation of a pharmaceutical composition, notably a dermatological composition, for the treatment of acne and of oily skins.

8. Use according to claim 7, characterised in that the above-mentioned Poria cocos extract is combined with at least one other active substance, preferably selected from the group consisting of a seboregulatory substance, an antiseptic substance, an anti-comedo substance, and an anti-inflammatory substance.

9. Use according to claim 8, characterised in that the above-mentioned seboregulatory substance is an extract of licorice, glycyrrhyza glabra.

10. Use according to claim 8, characterised in that the above-mentioned antiseptic substance is an anti-corynebacterium substance, such as hexamidine diisethionate, an isodon japonicus extract, clindamycin, or erythromycin.

11. Use according to claim 8, characterised in that the above-mentioned anti-comedo substance is selected from the group consisting of vitamin A acid, vitamin A and its derivatives such as the acetate, palmitate, propionate, and azelaic acid.

12. Use according to claim 8, characterised in that the above-mentioned anti-inflammatory substance is selected from the group consisting of ammonium glycyrrhizinate, glycyrrhetinic acid, α-bisabolol, tocopherol phosphate, and a corticoid.

13. Use according to one of claims 1 to 12, characterised in that it is an alcoholic extract, particularly a total ethanolic extract, or a hydro-alcoholic extract, such as a hydro-ethanolic or hydro-methanolic extract, of Poria cocos Wolf.

14. Use according to claim 1 to 13, characterised in that the concentration of Poria cocos extract is between 0.001 and 5% by weight with respect to the total weight of the final composition.

15. Use according to one of claims 1 to 14, characterised in that the above-mentioned extract is at least partially incorporated into hydrated lipidic lamellar phases or into liposomes.

16. A method of cosmetic treatment of oily skins, characterised in that a cosmetically effective quantity of an organic or hydro-organic extract of Poria cocos Wolf fungi, alone or included in a cosmetically acceptable excipient, is topically applied to the oily zones of the skin concerned.

17. The method of claim 16, characterised in that a cosmetic composition containing 0.001 to 5% by weight of the above-mentioned Poria cocos Wolf extract is applied.

18. The method of claim 16 or 17, characterised in that a cosmetic composition containing an alcoholic extract, in particular a total ethanolic extract, or a hydro-alcoholic extract such as a hydro-ethanolic or a hydro-methanolic extract, of Poria cocos Wolf fungi is applied.

19. The method according to one of claims 16 to 18, characterised in that the above-mentioned Poria cocos Wolf extract is at least partially incorporated into hydrated lipidic lamellar phases or into liposomes.

20. The method according to one of claims 16 to 19, characterised in that the above-mentioned Poria cocos extract is combined with at least one other active substance, preferably selected from the group consisting of a seboregulatory substance, an antiseptic substance, and an anti-comedo substance.

21. The method according to claim 20, characterised in that said substance is a seboregulatory substance constituting an extract of licorice, glycyrrhyza glabra.

22. The method according to claim 20, characterised in that said substance is a substance having antiseptic activity, preferably a substance having anti-corynebacterium activity such as an isodon japonicus extract.

23. The method according to claim 20, characterised in that said substance is a substance having anti-comedo activity selected from the group consisting of vitamin A acid and its derivatives such as the acetate, palmitate, propionate, and azelaic acid.

24. The method according to claim 20, characterised in that said substance is selected from the group consisting of ammonium glycyrrhizinate, glycyrrhetinic acid, $\alpha$-bisabolol, tocopherol phosphate.

**Patentansprüche**

1. Kosmetische Verwendung eines organischen oder wäßrig-organischen Extrakts von Poria cocos Wolf-Pilzen als Wirkstoff für die Pflege von fettiger Haut oder von Haut mit Akneneigung.

2. Verwendung nach Anspruch 1, dadurch gekennzeichnet, daß der Extrakt von Poria cocos mit mindestens einem anderen Wirkstoff, vorzugsweise ausgewählt aus der Gruppe, bestehend aus einer die Talgproduktion regulierenden Substanz, einer antiseptischen Substanz, einer gegen Mitesser wirksamen Substanz, kombiniert wird.

3. Verwendung nach Anspruch 2, dadurch gekennzeichnet, daß die Substanz eine die Talgproduktion regulierende Substanz, vorzugsweise ein Extrakt von Süßholz Glycyrrhyza glabra, ist.

4. Verwendung nach Anspruch 2, dadurch gekennzeichnet, daß die Substanz eine Substanz mit antiseptischer Wirkung, vorzugsweise eine Substanz mit einer Aktivität gegen Corynebakterien, wie ein Extrakt von Isodon japonicus, ist.

5. Verwendung nach Anspruch 2, dadurch gekennzeichnet, daß die Substanz eine gegen Mitesser wirksame Substanz, ausgewählt aus der Gruppe, bestehend aus Vitamin A-Säure, Vitamin A und seinen Derivaten, wie dem Acetat, Palmitat, Propionat, und Azelainsäure, ist.

6. Verwendung nach Anspruch 2, dadurch gekennzeichnet, daß die Substanz ausgewählt wird aus der Gruppe, bestehend aus Ammoniumglycyrrhizinat, Glycyrrhetinsäure, $\alpha$-Bisabolol, Tocopherolphosphat.

7. Verwendung eines organischen oder wäßrig-organischen Extrakts von Poria cocos Wolf-Pilzen für die Herstellung einer pharmazeutischen, insbesondere dermatologischen Zusammensetzung für die Behandlung von Akne und fettiger Haut.

8. Verwendung nach Anspruch 7, dadurch gekennzeichnet, daß der Extrakt von Poria cocos mit mindestens einem anderen Wirkstoff, vorzugsweise ausgewählt aus der Gruppe, bestehend aus einer die Talgproduktion regulierenden Substanz, einer antiseptischen Substanz, einer gegen Mitesser wirksamen Substanz, einer entzündungshemmenden Substanz, kombiniert wird.

9. Verwendung nach Anspruch 8, dadurch gekennzeichnet, daß die die Talgproduktion regulierende Substanz ein Extrakt von Süßholz Glycyrrhyza glabra ist.

10. Verwendung nach Anspruch 8, dadurch gekennzeichnet, daß die antiseptische Substanz eine gegen Corynebakterien wirksame Substanz, wie Hexamidindiisethionat, ein Extrakt von Isodon japonicus, Clindamycin oder Erythromycin, ist.

11. Verwendung nach Anspruch 8, dadurch gekenzeichnet, daß die gegen Mitesser wirksame Substanz aus der Gruppe, bestehend aus Vitamin A-Säure, Vitamin A und seinen Derivaten, wie dem Acetat, Palmitat, Propionat, und Azelainsäure, ausgewählt wird.

12. Verwendung nach Anspruch 8, dadurch gekennzeichnet, daß die entzündungshemmende Substanz aus der Gruppe, bestehend aus Ammoniumglycyrrhizinat, Glycyrrhetinsäure, $\alpha$-Bisabolol, Tocopherolphosphat und einem Corticoid, ausgewählt wird.

13. Verwendung nach einem der Ansprüche 1 bis 12, dadurch gekennzeichnet, daß es sich um einen alkoholischen Extrakt, insbesondere einen mittels absolutem Ethanol erhaltenen Extrakt, oder einen wäßrig-alkoholischen Extrakt, wie Wasser-Ethanol- oder Wasser-Methanol-Extrakt, von Poria cocos Wolf handelt.

14. Verwendung nach Anspruch 1 bis 13, dadurch gekennzeichnet, daß die Konzentration an Poria cocos-Extrakt zwischen 0,001 und 5 Gew.-% bezogen auf das Gesamtgewicht der endgültigen Zusammensetzung liegt.

15. Verwendung nach einem der Ansprüche 1 bis 14, dadurch gekennzeichnet, daß der Extrakt zumindest teilweise in hydratisierte lamellare Lipidphasen oder in Liposome eingebracht ist.

16. Verfahren zur kosmetischen Behandlung von fettiger Haut, dadurch gekennzeichnet, daß man auf die betreffenden fettigen Zonen der Haut topisch eine kosmetisch wirksame Menge eines organischen oder wäßrig-organischen Extrakts von Poria cocos Wolf-Pilzen allein oder eingebracht in einen kosmetisch annehmbaren Trägerstoff aufträgt.

17. Verfahren nach Anspruch 16, dadurch gekennzeichnet, daß man eine kosmetische Zusammensetzung, die 0,001 bis 5 Gew.-% des Extrakts von Poria cocos Wolf enthält, aufträgt.

18. Verfahren nach Anspruch 16 oder 17, dadurch gekennzeichnet, daß man eine kosmetische Zusammensetzung aufträgt, die einen alkoholischen Extrakt, insbesondere einen mittels absolutem Ethanol erhaltenen Extrakt, oder einen wäßrig-alkoholischen Extrakt, wie Wasser-Ethanol- oder Wasser-Methanol-Extrakt, von Poria cocos Wolf-Pilzen enthält.

19. Verfahren nach einem der Ansprüche 16 bis 18, dadurch gekennzeichnet, daß der Extrakt von Poria cocos Wolf zumindest teilweise in hydratisierte lamellare Lipidphasen oder in Liposome eingebracht ist.

20. Verfahren nach einem der Ansprüche 16 bis 19, dadurch gekennzeichnet, daß der Extrakt von Poria cocos mit mindestens einem anderen Wirkstoff, vorzugsweise ausgewählt aus der Gruppe, bestehend aus einer die Talgproduktion regulierenden Substanz, einer antiseptischen Substanz, einer gegen Mitesser wirksamen Substanz, kombiniert wird.

21. Verfahren nach Anspruch 20, dadurch gekennzeichnet, daß die Substanz eine die Talgproduktion regulierende Substanz, die einen Extrakt von Süßholz Glycyrrhyza glabra darstellt, ist.

22. Verfahren nach Anspruch 20, dadurch gekennzeichnet, daß die Substanz eine Substanz mit antiseptischer Wirkung, vorzugsweise eine gegen Corynebakterien wirksame Substanz, wie ein Extrakt von Isodon japonicus, ist.

23. Verfahren nach Anspruch 20, dadurch gekennzeichnet, daß die Substanz eine gegen Mitesser wirksame Substanz, ausgewählt aus der Gruppe, bestehend aus Vitamin A-Säure und ihren Derivaten, wie dem Acetat, Palmitat oder dem Propionat, und Azelainsäure, ist.

**24.** Verfahren nach Anspruch 20, dadurch gekennzeichnet, daß die Substanz ausgewählt wird aus der Gruppe, bestehend aus Ammoniumglycyrrhizinat, Glycyrrhetinsäure, α-Bisabolol, Tocopherolphosphat.